(19) Europäisches Patentamt
European Patent Office
Office européen des brevets

(11) Veröffentlichungsnummer: **0 007 616**
**B1**

(12) # EUROPÄISCHE PATENTSCHRIFT

(45) Veröffentlichungstag der Patentschrift: **12.05.82**

(51) Int. Cl.³: **C 07 D 277/64,** A 61 K 31/425

(21) Anmeldenummer: **79102620.6**

(22) Anmeldetag: **24.07.79**

(54) **Isothiocyanobenzthiazolderivat, Verfahren zu seiner Herstellung und seine Anwendung in anthelmintischen Mitteln.**

(30) Priorität: **27.07.78 CH 8082/78**

(43) Veröffentlichungstag der Anmeldung: **06.02.80 Patentblatt 80/3**

(45) Bekanntmachung des Hinweises auf die Patenterteilung: **12.05.82 Patentblatt 82/19**

(84) Benannte Vertragsstaaten: **BE CH DE FR GB IT NL**

(56) Entgegenhaltungen:
**1. CA-A-565 164**
**2. CH-A-506 321**
**3. CH-A-585 214**
**4. CH-A-587 837**

(73) Patentinhaber: **CIBA-GEIGY AG, Patentabteilung Postfach, CH-4002 Basel (CH)**

(72) Erfinder: **Boray, Joseph Coleman, Dr., 21 Bogota Avenue, Neutral Bay, N.S.W. 2089 (AU)**
Erfinder: **Gallay, Jean Jacques, Dr., Blumenrain 19, 4465 Magden (AU)**
Erfinder: **Sarasin, Gedeon, Dr., Chrischonastrasse 31, 4058 Basel (AU)**

(74) Vertreter: **Zumstein sen., Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 616 B1

BUNDESDRUCKEREI BERLIN

Isothiocyanobenzthiazolderivat, Verfahren zu seiner Herstellung und seine Anwendung in anthelmintischen Mitteln

Die vorliegende Erfindung betrifft das Isothiocyanobenzthiazol-Derivat der Formel I

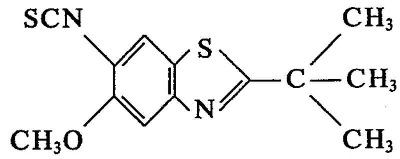

(I)

einschließlich seiner für Warmblüter nicht-toxischen Salze, sowie Mittel enthaltend diese Verbindung oder eines ihrer Salze als Wirkstoff und die Verwendung dieser Verbindung oder eines ihrer Salze zur Bekämpfung von Leberegeln (Fasciola hepatica) in Schafen.

Bereits in den schweizerischen Patentschriften Nr. 566 321 und Nr. 565 164 sowie Nr. 585 214 und Nr. 587 837 werden Gruppen von Isothiocyanobenzthiazolen beschrieben, in welchen die erfindungsgemäße Verbindung der Formel I zwar in allgemeiner Form mitumfaßt ist, jedoch nicht als Einzelverbindung spezifisch offenbart wird.

In den beiden zuerst genannten Patentschriften wird vorgeschlagen, diese Isothiocyanobenzthiazole zur Bekämpfung von Helminthen in Nutztieren einzusetzen, wobei unter »Helminthen« Nematoden, Cestoden und Trematoden zu verstehen sind. In der Beschreibung dieser Patente wird die Wirksamkeit einiger der offenbarten Verbindungen gegen einzelne der genannten Parasiten mit einer Ausnahme lediglich an Labortieren wie Ratten und Mäusen belegt. Die Ausnahme bezieht sich auf die Bekämpfung von Ascaridia galli in Hühnern. Prüfungsergebnisse von expressis verbis genannten Verbindungen hinsichtlich ihrer Wirksamkeit gegen Leberegel (Fasciola hepatica) betreffen jedoch ausnahmslos das Labortier (Ratte) als Wirtsorganismus.

Die beiden zuletzt genannten Patentschriften geben für eine Gruppe dieser Isothiocyanobenzthiazole zu der den umfaßten Verbindungen zukommenden breiten anthelmintischen Wirksamkeit als spezielles Anwendungsgebiet die Bekämpfung des im distalen Darmbereich parasitierenden Nematoden Oesophagostomum columbianum an.

Das Auftreten der gefährlichen und weit verbreiteten Fasciolose führt zu verringertem Wachstum, zu verminderter Leistung und sogar zu Todesfällen bei den befallenen Tieren und dadurch zu großen Schäden und Ertragseinbußen, insbesondere in der Schafzucht. Eine Bekämpfung und Vorbeugung der Schaffasciolose gilt demzufolge als vordringliche Aufgabe, um derartige, vor allem volkswirtschaftlich ins Gewicht fallende Ausfälle in der tierischen Produktion zu vermeiden.

Trotz zahlreicher bekannter Präparate auf dem Gebiet der Helminthenbekämpfung ist es wegen des breiten Spektrums der unter den Begriff »Helminthen) fallenden Parasiten bisher nicht gelungen, einen Wirkstoff zu entwickeln, der neben einer guten Wirkung gegen Nematoden und Cestoden gleichzeitig eine herausragende Aktivität gegen Leberegel in Schafen aufweist und damit eine erfolgreiche Bekämpfung der Fasciolose gewährleisten kann. Ferner hat sich gezeigt, daß die bisher vorgeschlagenen Mittel gegen Leberegel oft erhebliche toxische Nebenwirkungen besitzen, was einer Anwendung dieser Mittel aufgrund ihres ungünstigen therapeutischen Index' ohnehin enge Grenzen setzt.

So hat sich auch für die in den vier genannten Patentschriften spezifisch offenbarten und der erfindungsgemäßen Verbindung der Formel I strukturell nahestehenden Verbindungen eine Brauchbarkeit auf dem Gebiet der Leberegelbekämpfung im Nutztier, beispielsweise in Schafen in der Praxis nicht nachweisen lassen, obwohl die mit Labortieren erzielten Ergebnissen erfolgversprechend waren.

Es ist daher als besonders überraschend anzusehen, daß eine einzelne der in den genannten Patentschriften allgemein umfaßten Verbindungen, die jedoch nicht spezifisch offenbart ist, nämlich die erfindungsgemäße Verbindung der Formel I, neben allgemein guten anthelmintischen Eigenschaften eine ausgezeichnete Wirksamkeit gegen Leberegel in Schafen aufweist. Hinzu kommt, daß die erfindungsgemäße Verbindung einen außerordentlich günstigen therapeutischen Index (maximale Toleranzdosis/minimale Effektivdosis) von ca. 13 besitzt und demzufolge besonders für Therapien auf breiter Basis und aller Altersstufen der Schafe geeignet ist.

Als für den Warmblüter nichttoxische Salze des Isothiocyanobenzthiazols der Formel I kommen Additionsverbindungen mit anorganischen oder organischen Säuren, vorzugsweise stärkeren Säuren in Frage. Beispiele sind Chlorwasserstoffsäure, Bromwasserstoffsäure, Schwefelsäure, Phosphorsäure, Essigsäure, Adipinsäure, Maleinsäure, Weinsäure, Milchsäure, Citronensäure, Glutaminsäure, Aconitsäure, Sulfaminsäure, Methansulfonsäure, p-Toluolsulfonsäure.

Der erfindungsgemäße Wirkstoff kann den Tieren sowohl als Einzeldosis, wie auch wiederholt verabreicht werden, wobei die einzelnen Gaben je nach Tierart vorzugsweise zwischen 5 und 100 mg pro kg Körpergewicht betragen. Durch eine protrahierte Verabreichung wird in manchen Fällen eine bessere Wirkung erzielt oder eine geringere Gesamtdosis angewendet. Der Wirkstoff und ihn

enthaltende Gemische können auch dem Futter zugesetzt werden. Das Fertigfutter enthält den Wirkstoff der Formel I vorzugsweise in einer Konzentration von etwa 0,05 bis 1 Gew.-%.

Der neue Wirkstoff kann als Mittel, z. B. in Form von Lösungen, Emulsionen, Suspensionen (Drenchs), Pulvern, Tabletten, Bolussen und Kapseln peroral oder intra-ruminal den Tieren verabreicht werden. Zur Bereitung der oben angeführten Applikationsformen dienen beispielsweise übliche feste Trägerstoffe, wie Kaolin, Talkum, Bentonit, Kochsalz, Calciumphosphat, Kohlehydrat, Cellulosepulver, Baumwollsaatmehl, Carbowaxe, Gelatine, oder Flüssigkeiten wie Wasser, gewünschtenfalls unter Zusatz von oberflächenaktiven Stoffen, wie ionischen oder nichtionischen Dispersionsmittel, sowie Ölen und anderen für den tierischen Organismus unschädlichen Lösungs- und Verdünnungsmitteln. Bei der Anwendung des Wirkstoffes in anthelmintischen Mitteln in Form von Futterkonzentraten dienen als Trägerstoffe zum Beispiel Leistungsfutter, Futtergetreide oder Proteinkonzentrate.

Den beschriebenen erfindungsgemäßen Mitteln lassen sich andere biozide Wirkstoffe, wie z. B. Pestizide, beimischen. So können die Mittel außer der genannten Verbindung der Formel I zum Beispiel Fungizide, Bakterizide, Fungistatika, Bakteriostatika, Ektoparasitizide oder andere Wirkstoffe zur Verbreiterung des Wirkungsspektrums enthalten.

Das Isothiocyanobenzthiazol der Formel I läßt sich nach bekannten Verfahren, wie beispielsweise in der schweizerischen Patentschrift Nr. 566 321 beschrieben, herstellen, indem man das Aminobenzthiazol der Formel II

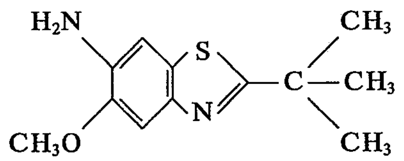

(II)

mit einem Thiokohlensäure-Derivat der Formel

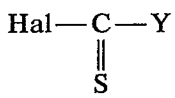

in der Hal Chlor oder Brom und Y Chlor, Brom oder eine Dialkylaminogruppe bedeuten, in einem inerten Lösungs- oder Verdünnungsmittel bei Temperaturen von −20° bis +100° C, vorzugsweise −10° bis +30° C, umsetzt.

### Beispiel

Herstellung von 6-Isothiocyano-5-methoxy-2-tert.-butyl-benzthiazol

32,0 g 6-Amino-5-methoxy-2-tert.-butyl-benzthiazol werden in 200 ml Dioxan gelöst, bei 10−15° C gerührt und mit einem Gemisch von 25 ml Thiophosgen und 25 ml Dioxan unter Rühren tropfenweise versetzt, bei gleicher Temperatur 18 Stunden weitergerührt, dann auf 400 ml mit Wasser verdünnt und eine weitere Stunde gerührt. Der gebildete Niederschlag wird abgesaugt, mit Wasser gewaschen und feucht in 500 ml heißem Acetonitril gelöst, mit 100 ml Wasser verdünnt und abgekühlt. Das auskristallisierte Produkt wird abgesaugt und getrocknet. Man erhält 18 bis 25 g 6-Isothiocyano-5-methoxy-2-tert.-butyl-benzthiazol vom Smp. 73−74° C.

Die anthelmintische Wirksamkeit wird anhand folgender Versuche demonstriert:

### 1. Versuch an mit Nematoden wie Haemonchus contortus und Trichostrongylus colubriformis infizierten Schafen

Der Wirkstoff wird in Form einer Suspension mittels einer Magensonde oder durch Injektion in den Pansen Schafen verabreicht, die mit Nematoden wie Haemonchus contortus und Trichostrongylus colubriformis künstlich infiziert worden sind. Pro Versuch resp. Dosis werden 3 Tiere verwendet. Jedes Schaf wird mit nur einer einzigen Dosis behandelt.

Eine erste Evaluierung erfolgt dadurch, daß die Anzahl der vor und nach der Behandlung im Kot der Schafe ausgeschiedenen Wurmeiern verglichen werden.

Sieben bis zehn Tage nach der Behandlung werden die Schafe getötet und seziert. Die Auswertung erfolgt durch Auszählung der nach der Behandlung im Darm zurück gebliebenen Würmer. Gleichzeitig und gleichartig infizierte aber unbehandelte Schafe dienen als Kontrolle resp. Vergleich.

2. Versuch an mit Cestoden wie Moniezia benedeni infizierten Schafen

Der Wirkstoff wird in Form einer Suspension mittels einer Magensonde oder durch Injektion in den Pansen Schafen verabreicht, die mit Cestoden wie Moniezia benedeni infiziert sind. Pro Versuch resp. pro Dosis werden 3 Tiere verwendet. Jedes Schaf wird mit nur einer einzigen Dosis behandelt.

Sieben bis zehn Tage nach der Behandlung werden die Schafe getötet und seziert. Die Auswertung erfolgt durch Auszählung der nach der Behandlung im Darm zurück gebliebenen Würmer. Gleichzeitig und gleichartig infizierte aber unbehandelte Schafe dienen als Kontrolle resp. Vergleich.

3. Versuch an mit Fasciola hepatica infizierten Schafen

Der Wirkstoff wird in Form einer Suspension mittels einer Magensonde oder durch Injektion in den Pansen Schafen verabreicht, die mit Fasciola hepatica künstlich infiziert worden sind. Pro Versuch resp. Dosis werden 3 Tiere verwendet. Jedes Tier wird mit nur einer einzigen Dosis behandelt.

Eine erste Evaluierung erfolgt dadurch, daß die Anzahl der vor und nach der Behandlung im Kot der Schafe ausgeschiedenen Wurmeier verglichen werden.

Drei bis vier Wochen nach der Behandlung werden die Schafe getötet und seziert. Die Auswertung erfolgt durch das Auszählen der nach der Behandlung in den Gallengängen zurück gebliebenen Leberegel. Gleichzeitig und gleichartig infizierte aber unbehandelte Schafe dienen als Kontrolle resp. Vergleich. Der Unterschied der in den beiden Gruppen festgestellten Anzahl von Leberegeln ergibt den Wirkungsgrad des geprüften Wirkstoffs.

Die Verträglichkeit wird anhand folgender Versuche demonstriert.

Verträglichkeit beim Schaf

Die Wirkstoffe werden in Form einer Suspension mittels einer Magensonde oder durch Injektion in den Pansen den Versuchstieren verabreicht. Pro Versuch resp. pro Dosis werden 3–5 Schafe eingesetzt. Jedes Tier wird mit nur einer einzigen Dosis behandelt.

Nach der Behandlung werden die Schafe während 14 Tagen unter Beobachtung gehalten. In regelmäßigen Abständen werden die Schafe gewogen. Gleichzeitig werden allfällig auftretende, klinische Symtome und Todesfälle registriert. Als Kontrolle werden gleichartige (bezüglich Rasse, Alter, Gewicht), nicht behandelte Schafe zusammen mit den Versuchstieren gehalten. Bei diesen Kontroll-Schafen werden dieselben Parameter registriert wie bei den behandelten Tieren.

Versuchsergebnisse (Fasciola hepatica-Therapie beim Schaf)

| Nr. | Verbindung | Dosis | |
|---|---|---|---|
| | | MED*) | MTD*) |
| | | in mg/kg Körpergewicht | |
| 1 | 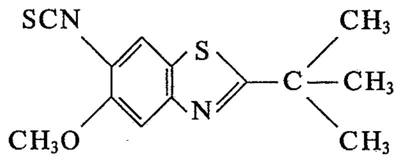 | 25 | 320 |
| 2 | 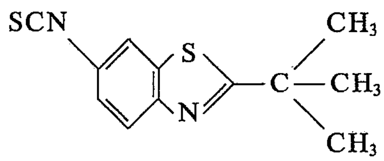 | 100 | 200 |
| 3 | 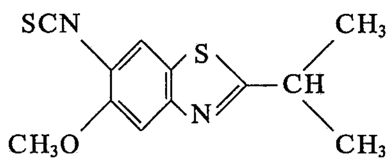 | >100 | 200 |
| 4 | 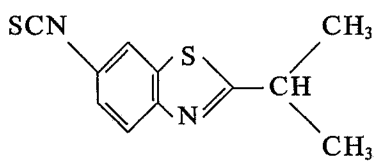 | >200 | 200 |

*) MED = minimale effektive Dosis.
*) MED = maximale tolerierte Dosis.

Die bei der Verbindung der Formel I minimale effektive Dosis von 25 mg/kg (siehe Tabelle) ist beim Schaf außer gegen Fasciola hepatica auch gegen Nematoden wie Haemonchus concortus, Trichostrongylus colubriformis und Ostertagia circumcincta und gegen Cestoden wie Moniezia benedeni wirksam.

**Patentansprüche**

1. Verbindung der Formel I

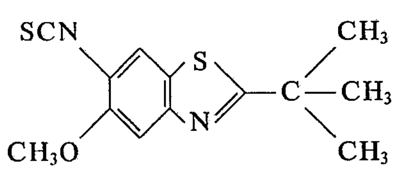 (I)

und ihre für Warmblüter nicht-toxischen Säureadditionssalze.

2. Anthelmintische Mittel enthaltend als Wirkstoff die Verbindung oder eines ihrer Salze gemäß Anspruch 1 zusammen mit Trägerstoffen und/oder Verteilungsmitteln.

3. Verfahren zur Herstellung der Verbindung der Formel I gemäß Anspruch 1, dadurch gekennzeichnet, daß man das Aminobenzthiazol der Formel II

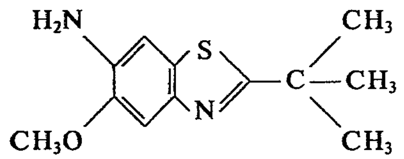

(II)

mit einem Thiokohlensäure-Derivat der Formel

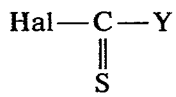

in der Hal Chlor oder Brom und Y Chlor, Brom oder eine Dialkylaminogruppe bedeuten, in einem inerten Lösungs- oder Verdünnungsmittel bei Temperaturen von −20° bis +100° C umsetzt.

4. Verbindung der Formel I oder eines ihrer Salze gemäß Anspruch 1 zur Verwendung bei der Bekämpfung von Helminthen.

5. Verbindung der Formel I oder eines ihrer Salze gemäß Anspruch 1 zur Verwendung bei der Bekämpfung von Leberegeln (Fasciola hepatica), Nematoden und Cestoden in Wiederkäuern, insbesondere in Schafen.

6. Verbindung der Formel I oder eines ihrer Salze gemäß Anspruch 1 zur Verwendung bei der Bekämpfung von Leberegeln (Fasciola hepatica) in Wiederkäuern, insbesondere in Schafen.

7. Anthelmintische Mittel gemäß Anspruch 2 zur Verwendung bei der Bekämpfung von Leberegeln (Fasciola hepatica), Nematoden und Cestoden in Wiederkäuern, insbesondere in Schafen.

8. Anthelmintische Mittel gemäß Anspruch 2 zur Verwendung bei der Bekämpfung von Leberegeln (Fasciola hepatica) in Wiederkäuern, insbesondere in Schafen.

9. Die nach dem Verfahren von Anspruch 3 erhältlichen Verbindungen.

## Claims

1. A compound of the formula I

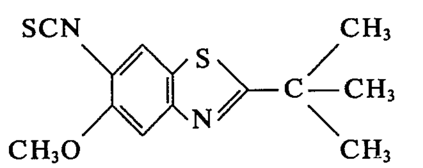

(I)

and acid addition salts thereof nontoxic to warm-blooded animals.

2. An anthelmintic composition containing as active substance the compound or one of the salts thereof according to Claim 1, together with carriers and/or distributing agents.

3. A process for producing the compound of the formula I according to Claim 1, which process comprises reacting the aminobenzothiazole of the formula II

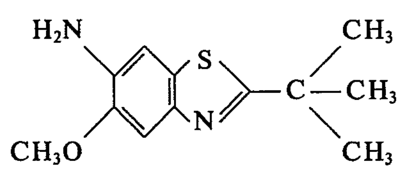

(II)

with a thiocarbonic acid derivative of the formula

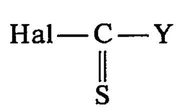

in which »Hal« is chlorine or bromine, and Y is chlorine, bromine or a dialkylamino group, in an inert solvent or diluent, at temperatures of −20° to +100° C.

4. A compound of the formula I or a salt thereof according to Claim 1 for use in combating helminthes.

5. A compound of the formula I or a salt thereof according to Claim 1 for use in combating liver flukes (Fasciola hepatica), nematodes and cestodes in ruminants, particularly in sheep.

6. A compound of the formula I or a salt thereof according to Claim 1 for use in combating liver flukes (fasciola hepatica) in ruminants, particularly in sheep.

7. An anthelmintic composition according to Claim 2 for use in combating liver flukes (Fasciola hepatica), nematodes and cestodes in ruminants, particularly in sheep.

8. An anthelmintic composition according to Claim 2 for use in combating liver flukes (Fasciola hepatica) in ruminants, particularly in sheep.

9. The compounds obtainable by the process of Claim 3.

**Revendications**

1. Composé de formule I:

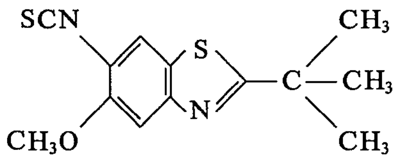

(I)

et ses sels formés par addition avec des acides non toxiques pour les individus à sang chaud.

2. Agent anti-helmintique contenant en tant que substance active le composé ou l'un de ses sels selon la revendication 1 avec des véhicules et/ou des agents distribuants.

3. Procédé de préparation du composé de formule I selon la revendication 1, caractérisé en ce que l'on fait réagir l'aminobenzothiazole de formule II:

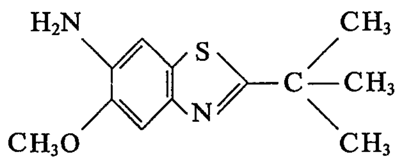

(II)

avec un dérivé de l'acide thiocarbonique de formule

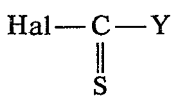

dans laquelle Hal représente le chlore ou le brome et Y représente le chlore, le brome ou un groupe dialkylamino, dans un solvant ou diluant inerte à des températures allant de $-20$ à $+100°$ C.

4. Composé de formule I ou l'un de ses sels selon la revendication 1, pour l'utilisation dans la lutte contre les helminthes.

5. Composé de formule I ou l'un de ses sels selon la revendication 1, pour l'utilisation dans la lutte contre la douve (Fasciola hepatica), les nématodes et les cestodes chez les ruminants, en particulier, chez les moutons.

6. Composé de formule I ou l'un de ses sels selon la revendication 1, pour l'utilisation dans la lutte contre la douve (Fasciola hepatica) chez les ruminants, en particulier les mounts.

7. Agent anti-helmintique selon la revendication 2, pour l'utilisation dans la lutte contre la douve (Fasciola hepatica), les nématodes et les cestodes chez les ruminants, en particulier les moutons.

8. Agent anti-helmintique selon la revendication 2, pour l'utilisation dans la lutte contre la douve (Fasciola hepatica) chez les ruminants, en particulier les moutons.

9. Les composés susceptibles d'être obtenus par le procédé selon la revendication 3.